Europäisches Patentamt

European Patent Office

Office européen des brevets

(19)

(11) Publication number: **0 315 247 B1**

## (12) EUROPEAN PATENT SPECIFICATION

(45) Date of publication of patent specification: **15.04.92**

(51) Int. Cl.⁵: **C07D 301/12**, C07D 303/04

(21) Application number: **88202369.0**

(22) Date of filing: **25.10.88**

(54) **Process for the production of epoxy derivatives with cis-configuration, by starting from mixtures of the corresponding olefinic stereoisomers.**

(30) Priority: **02.11.87 IT 2248487**

(43) Date of publication of application:
**10.05.89 Bulletin 89/19**

(45) Publication of the grant of the patent:
**15.04.92 Bulletin 92/16**

(84) Designated Contracting States:
**AT BE CH DE ES FR GB GR LI LU NL SE**

(56) References cited:
**EP-A- 0 100 119**

**TETRAHEDRON LETTERS, vol. 27, no. 6, 1986, pages 707-710, Pergamon Press Ltd, GB; D. PRAT et al.: "Epoxidations with 30% hydrogen peroxide catalyzed by tungstic acid in buffered media"**

(73) Proprietor: **ENIRICERCHE S.p.A.**
**Corso Venezia 16**
**I-20121 Milan(IT)**

(72) Inventor: **Clerici, Mario Gabriele**
**Via Europa 34**
**I-20097 San Donato Milanese Milan(IT)**
Inventor: **Bellussi, Giuseppe**
**Via Alberto Scoto 44**
**I-20199 Piacenza(IT)**

(74) Representative: **Roggero, Sergio et al**
**Ing. Barzanò & Zanardo Milano S.p.A. Via Borgonuovo 10**
**I-20121 Milano(IT)**

Rank Xerox (UK) Business Services

EP 0 315 247 B1

## Description

This invention relates to a process for preparing "cis" epoxy derivatives of a mixture of "cis" and "trans" isomers of unsubstituted alkenes by epoxidizing such a mixture with hydrogen peroxide.

EP-A-100 119 teaches that epoxidation of olefine compounds with hydrogen peroxide can be efficiently catalyzed by titanium-containing synthetic zeolites.

When the starting compound is a mixture of "cis" and "trans" isomers of unsubstituted alkenes, the final epoxidation product is a mixture of the corresponding isomers.

This inventions has as its objective the preparation of predominantly "cis" isomers of the final epoxidation product, the amounts of the "trans"isomers being extremely reduced.

To solve this problem, the present invention provides a process for preparing "cis" epoxy derivatives from a mixture of "cis" and "trans" isomers of unsubstituted alkenes by epoxidizing such a mixture with hydrogen peroxide, or with a hydrogen-peroxide-evolving compound, in the presence of an epoxidation catalyst, characterized in that the epoxidation catalyst is a synthetic zeolite having the formula:

$$x.TiO_2.(1-x)SiO_2$$

wherein $x$ is from 0,001 and 0,04, and in that the process is carried out with an amount of hydrogen peroxide lower than, or equal to the molar amount of the "cis" isomer of the starting isomer mixture, whereby a predominantly "cis" epoxy derivative is obtained and separated from the unreacted predominantly "trans" unsubstituted alkenes, these latter are subjected to low temperature isomerization until the equilibrium composition is obtained, and the thus isomerized alkenes are recycled to the epoxidation environment.

Preferably, the amount of hydrogen peroxide is from 0,3 mol to 1,0 mol per mol of the "cis"isomeric starting unsubstituted alkenes.

The epoxidation temperature is from -20°C to 100°C, the preferred range being from 0°C to 70°C.

Hydrogen peroxide can be used as an aqueous solution, the concentration being preferably of from 10% to 70% by volume of $H_2O_2$.

As a general rule, the epoxidation reaction is carried out in a polar solvent medium selected from the alcohols, the glycols, the ketones, the ethers, the esters and water.

Exemplary starting unsubstituted alkenes are butene-2, pentene-2 and octene-2.

The catalyst based on titanium-containing zeolite can be prepared, for example, according to the teachings of IT-A-22608 A/82 and IT-A-20487 A/85.

In case of volatile olefinic products, the reaction can be carried out under pressures which may reach values as high as 5066,16 kPa (50 atmospheres).

The separation between the epoxy compounds obtained from the reaction and the unreacted olefinic compounds can be advantageously carried out by distillation. This operation, in case the products and/or the reactants have a high boiling point, can be carried out under reduced pressure.

The reaction of isomerization can be carried out at a temperature comprised within the range of from 0 to 400°C in the presence of isomerization catalysts known from the prior art, such as those based on possibly supported metals, or of either liquid or solid acids.

The reaction can take place in continuous manner according to the process flow scheme reported in the hereto attached drawing, wherein 1 is the duct through which the mixture of olefinic stereoisomer compounds is fed, 2 is the duct through which hydrogen peroxide is fed, 3 is the oxidation reactor, 4 is the column of separation of the unreacted olefinic compound which is sent to the reaction of isomerization 7 through the duct 6, whilst the epoxy product is sent through the line 8 to the column 12 of separation of the product 11 and of recovery of the solvent 9, which is recycled to the oxidation reactor. The unreacted olefinic compound after the isomerization is recycled to the feed through the line 10.

The above process scheme can be modified according to the starting products, the solvents, and so forth.

In particular, the solvent separation step can be eliminated if the process is carried out in the absence of said solvent, or must be modified, in case the solvent used in more volatile than the reactants and/or the products.

The following examples illustrate the invention without limiting it.

Example 1

A titanium-silicalite zeolite is prepared as follows: 4,160 g of tetra-ethyl-silicate is prepared in a pyrex-glass beaker, and to it first 137 g of tetra-ethyl-titanate, then 7,310 g of an aqueous solution at 25% by weight of tetrapropyl-ammonium hydroxide are added with stirring. The mixture is stirred for about 5 hours at 80°C, and then demineralized water is added up to an end volume of about 14 litres. The obtained mixture is charged to an autoclave, and is left 10 days at 175°C under its autogenous pressure. The crystalline solid which is discharged is washed, dried and calcined at 550°C. The X-rays and I.R. analyses show that the product obtained is titanium-silicalite.

To the so-prepared zeolite, the following are added: 370 g of tetra-ethyl-silicate with vigorous stirring, and 395 g of an aqueous solution of tetra-propyl-ammonium hydroxide at 12% by weight, and the resulting mixture is heated for 1 hour at 60°C; then, 1,620 g of demineralized water is added, and the resulting mixture is kept stirred for a further hour. A clear solution is thus obtained, in which 1000 g of the 4-zeolite obtained as described in the Example 7, is carefully dispersed.

The milky suspension resulting from the dispersion is fed to a spray-drier (a NIRO ATOMIZER disk-atomizer; temperature of entering air = 300°C; temperature of exiting air = 120°C; chamber diameter = 1.5 metres), with compact microspheres being obtained, which have an average diameter close to 20 $\mu$m.

The atomized product is charged to a muffle under an $N_2$ blanket and is heated up to 550°C. After a two-hour stay at that temperature under nitrogen, the atmosphere is gradually changed from $N_2$ to air, and the product is maintained for a further two hours at 550°C under air. The obtained product has the following chemical molar composition:

$0.02\ TiO_2 : SiO_2$

Example 2
------

To a jacketed reactor of 1 litre of capacity, equipped with a temperature-control system, mechanical stirring means and pressure control system, 300 g of methanol, 5.0 g of titanium-silicalite prepared as described in Example 1, and 37 g of butene (butene-1 = 10%; cis-2-butene = 43%; trans-2-butene = 47%) are charged.

To a supply vessel connected to the reactor, 13.5 g of 60%-$H_2O_2$ ($C_4H_8/H_2O_2$ = 2.8 by mol) is charged.

After regulating the temperature at the controlled value of 22°C, hydrogen peroxide is added to the suspension with strong stirring.

The disappearing of hydrogen peroxide is monitored by drawing samples of the reaction mixture, and submitting them to the iodometric analysis.

After 75 minutes, the conversion is of 98%. The quantitative analysis of the reaction products (g.l.c. on a 1.8-metre long column packed with Parapak PS, with benzene as the internal standard) shows the following composition of the products:
- cis-2,3-epoxy-butane : 0.222 mol
- trans-2,3-epoxy-butane : 0.007 mol
- 1,2-epoxy-butane : 0.004 mol
- solvolysis products : traces

Unreacted butene (23.93 g; cis-2-butene: 14.5%; trans-2-butene: 71.0%; 1-butene: 14.5%) is distilled off from the reaction mixture under reduced pressure, with it being recovered as the overhead stream. Collected butene is charged to a small-size autoclave equipped with magnetic-driven stirring means, together with 0.5 g of an isomerization catalyst constituted by palladium acetylacetonate supported on silica. The suspension is left at 40°C with stirring for 3 hours, and butene is then separated and recovered: 23.5 g (cis-2-butene: 39.3%; trans-2-butene: 56.8%; 1-butene: 3.9%).

With the initially seen modalities, the amount of 23.5 g of isomerized butenes is combined with the suspension coming from the first distillation. Furthermore, 7.1 g of $H_2O_2$ at 60% is added. The temperature of the reaction system is regulated at the value of 22°C with strong stirring, and 90 minutes later 99% of hydrogen peroxide has reacted.

The g.l.c. analysis of the reaction mixture shows that the reaction products have the following composition:
- cis-2,3-epoxy-butane : 0.339 mol
- trans-2,3-epoxy-butane : 0.010 mol
- 1,2-epoxy-butane : 0.0057 mol
- solvolysis products : 0.002 mol

The process can be repeated in a similar way, until the mixture of linear butenes is converted into an epoxide prevailingly constituted by the cis-isomer.

Example 3
------

The instant example is supplied for comparison purposes, but does not fall within the scope of the present invention.

The process is carried out by the same modalities as of Example 2. To the jacketed reactor, 300 g of methanol, 5.0 g of titanium-silicalite prepared as described in Example 1, and 37 g of butenes (butene-1: 10%; cis-2-butene: 43%; trans-2-butene: 47%) are charged.

To a supply vessel connected to the reactor, 37.0 g of 60%-$H_2O_2$ ($C_4H_8/H_2O_2$ = 1.0 by mol) is charged.

After regulating the temperature at the controlled value of 22°C, hydrogen peroxide is added to the suspension with strong stirring.

After 100 minutes, the conversion of $H_2O_2$ is of 96%.

The reaction products are constituted by:
- cis-2,3-epoxy-butane : 0.270 mol
- trans-2,3-epoxy-butane : 0.279 mol
- 1,2-epoxy-butane : 0.063 mol
- solvolysis products : 0.014 mol

Example 4
------

In a reactor similar to that of Example 2, and with the same modalities, 330 g of methanol, 4.5 g of titanium-silicalite prepared as in Example 1, 29 g of pentene-2 (35% cis-isomer, 65% trans-isomer) and 7.8 g of $H_2O_2$ at 60% ($C_5H_{10}/H_2O_2$ = 3.0 by mol) are reacted.

After 60 minutes at 25°C, the conversion of $H_2O_2$ is of 97%, with the following reaction products being formed:
- cis-2,3-epoxy-pentane : 0.126 mol
- trans-2,3-epoxy-pentane : 0.0066 mol

Unreacted pentene is separated at 25°C under reduced pressure from the reaction mixture, and is transferred to a small glass autoclave together with 1 g of an isomerization catalyst constituted by 5% Pd on silica. After being kept 1 hout at 80°C with stirring, the suspension is cooled and pentene is distilled off.

It is then combined with the starting suspension together with 4.5 g of $H_2O_2$ at 60%, and is allowed to react at 25°C in the same way as in the first cycle.

After 90 minutes, the conversion of $H_2O_2$ is of 96%, with the reaction products having the following composition:
- cis-2,3-epoxy-pentane : 0.197 mol
- trans-2,3-epoxy-pentane : 0.009 mol
- 1,2-epoxy-pentane : 0.0008 mol
- solvolysis products : 0.003 mol

In an analogous way, the isomerization, epoxidation cycles can be repeated, or fresh olefin can be charged in order to replace the reacted olefin.

Example 5

The instant example is supplied for comparison purposes, but does not fall within the scope of the invention.

In the same reactor as of Example 4, and with analogous modalities, 230 g of methanol, 4.5 g of titanium-silicalite prepared as described in Example 1, 29 g of pentene-2 (cis-isomer 35%, trans-isomer 65%) and 23.0 g of $H_2O_2$ at 60% ($C_5H_{10}/H_2O_2$ = 1.02) are reacted.

After 75 minutes at 28°C, the conversion of $H_2O_2$ is of 96%, and the composition of the reaction products is as follows:
- cis-2,3-epoxy-pentane : 0.135 mol
- trans-2,3-epoxy-pentane : 0.251 mol
- solvolysis products : 0.003 mol

Example 6

To a jacketed glass reactor equipped with a temperature-control system and magnetic-driven stirring means, 17.0 g of octene-2 (cis-isomer/trans-isomer = 40/60), 84 g of methanol, 2.83 g of $H_2O_2$ at 60% ($C_8H_{16}/H_2O_2$ = 3.0 by mol), and 1.3 g of titaniun-silicalite prepared as described in Example 1 are charged. Also added is 0.92 g of nonane as the internal standard. After 63 minutes of reaction at 44°C, the conversion of $H_2O_2$ reaches the value of 96%.

The analysis of the reaction products gives the following composition:
- cis-2,3-epoxy-octane : 0.044 mol
- trans-2,3-epoxy-octane : 0.007 mol
- solvolysis products : 0.0011 mol

Residual octene (11.6 g; 15% cis-isomer and 85% trans-isomer) can be recovered and isomerized to yield the cis/trans equilibrium composition, and reacted with a defect of $H_2O_2$ to produce again cis-2,3-octane-epoxide.

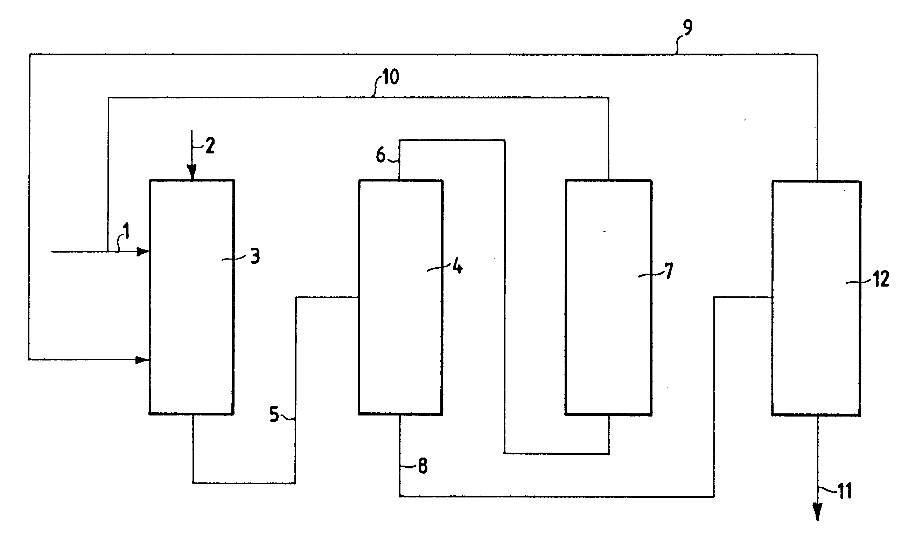

**Claims**

1. Process for preparing "cis" epoxy derivatives of a mixture of "cis" and "trans" isomers of unsubstituted alkenes by epoxidizing such a mixture with hydrogen peroxide, or with a hydrogen-peroxide-evolving compound, in the presence of an epoxidation catalyst, characterized in that the epoxidation catalyst is a synthetic zeolite having the formula:

   $$x.TiO_2.(1-x)SiO_2$$

   wherein **x** is from 0,0001 to 0,04 , and in that the process is carried out with an amount of hydrogen peroxide lower than, or equal to the molar amount of the "cis" isomer of the starting isomer mixture, whereby a product comprising predominantly "cis" epoxy derivative is obtained and separated from the unreacted predominantly "trans" unsubstituted alkenes, these latter are subjected to low temperature isomerization until the equilibrium composition is obtained, and the thus isomerized alkenes are recycled to the epoxidation environment.

2. Process according to Claim 1 , wherein the starting unsubstituted alkenes are selected from butene-2, pentene-2 and octene-2.

3. Process according to Claim 1, wherein the amount of hydrogen peroxide is from 0,3 mol to 1,0 mol per mol of the "cis"isomeric starting unsubstituted alkenes.

4. Process according to Claim 1 , wherein the epoxidation temperature is from -20°C to 100°C.

5. Process according to Claim 4, wherein the epoxidation temperature is from 0°C to 70°C.

6. Process according to Claim 1, wherein the epoxidation reaction is carried out in a polar solvent medium selected from the alcohols, the glycols, the ketones, the ethers, the esters and water.

7. Process according to Claim 1, wherein the epoxidation reaction is carried out under a pressure of from 101,3232 kPa to 5066,16 kPa (from 1 to 50 abs.atmospheres).

8. Process according to Claim 1, wherein the isomerization of the unreacted predominantly "trans" unsubstituted alkenes is carried out in the presence of a Pd-based catalyst.

**Revendications**

1. Procédé de préparation de dérivés cis-époxy d'un mélange d'isomères cis et trans d'alcènes non substitués, par époxydation d'un tel mélange à l'aide de peroxyde d'hydrogène ou d'un composé dégageant du peroxyde d'hydrogène, en présence d'un catalyseur d'époxydation, procédé caractérisé en ce que le catalyseur d'époxydation est une zéolithe synthétique de formule :

$$xTiO_2 . (1 - x) SiO_2$$

dans laquelle x vaut de 0,0001 à 0,04, et en ce que le procédé est effectué avec une quantité de peroxyde d'hydrogène inférieure ou égale à la quantité, exprimée en moles, d'isomère cis présente dans le mélange d'isomères de départ, grâce à quoi l'on obtient un produit contenant principalement du dérivé cis-époxy, produit que l'on sépare d'avec les alcènes non substitués n'ayant pas réagi, principalement trans, et ces derniers sont soumis à une isomérisation à basse température jusqu'à ce que l'on obtienne la composition d'équilibre, les alcènes ainsi isomérisés étant alors recyclés vers l'étape d'époxydation.

2. Procédé conforme à la revendication 1, dans lequel les alcènes non substitués de départ sont choisis parmi le butène-2, le pentène-2 et l'octène-2.

3. Procédé conforme à la revendication 1, dans lequel la quantité de peroxyde d'hydrogène vaut de 0,3 mole à 1,0 mole par mole d'isomères cis des alcènes non substitués de départ.

4. Procédé conforme à la revendication 1, dans lequel la température d'époxydation vaut de -20°C à 100°C.

5. Procédé conforme à la revendication 4, dans lequel la température d'époxydation vaut de 0°C à 70°C.

6. Procédé conforme à la revendication 1, dans lequel la réaction d'époxydation est effectuée dans un solvant polaire choisi parmi les alcools, les glycols, les cétones, les éthers, les esters et l'eau.

7. Procédé conforme à la revendication 1, dans lequel la réaction d'époxydation est effectuée sous une pression de 101,3232 kPa à 5066,16 kPa (de 1 à 50 atm abs).

8. Procédé conforme à la revendication 1, dans lequel l'isomérisation des alcènes non substitués n'ayant pas réagi, principalement trans, est effectuée en présence d'un catalyseur à base de palladium.

**Patentansprüche**

1. Verfahren zur Herstellung von cis-Epoxyderivaten eines Gemisches aus cis-und trans-Isomeren von unsubstituierten Alkenen durch Epoxidieren eines solchen Gemisches mit Wasserstoffperoxid oder mit einer Wasserstoffperoxidfreisetzenden Verbindung in Gegenwart eines Epoxidationskatalysators, dadurch gekennzeichnet, daß der Epoxidationskatalysator ein synthetischer Zeolith mit der Formel:

$$x.TiO_2 . (1-x)SiO_2$$

ist, worin x einen Wert von 0,0001 bis 0,04 aufweist, und daß das Verfahren mit einer Wasserstoffperoxidmenge ausgeführt wird, die kleiner als oder gleich der Molmenge des cis-Isomers des eingesetzten Isomerengemisches ist, wodurch ein überwiegend das cis-Epoxyderivat enthaltendes Produkt erhalten und von den nicht umgesetzten, überwiegend trans-unsubstituierten Alkenen abgetrennt wird, wobei die letztgenannten einer Niedertemperaturisomerisierung unterworfen werden, bis die Gleichgewichtszusammensetzung erhalten wird, und die solcherart isomerisierten Alkene zum Epoxidationsbereich zurückgeführt werden.

2. Verfahren nach Anspruch 1, worin die eingesetzten unsubstiuierten Alkene unter Buten-2, Penten-2 und Octen-2 ausgewählt werden.

3. Verfahren nach Anspruch 1, worin die Wasserstoffperoxidmenge von 0,3 Mol bis 1,0 Mol je Mol der eingesetzten unsubstituierten cis-Alkenisomeren beträgt.

4.  Verfahren nach Anspruch 1, worin die Epoxidationstemperatur von -20°C bis 100°C beträgt.

5.  Verfahren nach Anspruch 4, worin die Epoxidationstemperatur von 0°C bis 70°C beträgt.

6.  Verfahren nach Anspruch 1, worin die Epoxidationsreaktion in einem polaren Lösungsmittelmedium, ausgewahlt unter Alkoholen, Glykolen, Ketonen, Ethern, Estern und Wasser, ausgeführt wird.

7.  Verfahren nach Anspruch 1, worin die Epoxidationsreaktion unter einem Druck von 101,3232 kPa bis 5066,16 kPa (1 bis 50 Atmosphären absolut) ausgeführt wird.

8.  Verfahren nach Anspruch 1, worin die Isomerisierung der nicht umgesetzten, überwiegend trans-unsubstituierten Alkene in Gegenwart eines Katalysators auf Palladiumbasis ausgeführt wird.